# EUROPÄISCHE PATENTSCHRIFT

(11) **EP 1 397 208 B1**
(45) Veröffentlichungstag und Bekanntmachung des Hinweises auf die Patenterteilung: **22.03.2006**
(21) Anmeldenummer: 02750940.5
(22) Anmeldetag: 16.05.2002
(51) Int. Cl.: B01J 23/755, B01J 21/06, B01J 35/10, B01J 37/03, C07C 209/36

(54) **NI/TIO2-HYDRIERKATALYSATOR**
NI/TIO2 HYDROGENATION CATALYST
CATALYSEUR D'HYDROGENATION NI/TIO2

(30) Priorität: 21.05.2001 DE 10124600
(43) Veröffentlichungstag der Anmeldung: 17.03.2004
(73) Patentinhaber: BASF Aktiengesellschaft, 67056 Ludwigshafen (DE)
(72) Erfinder: VANOPPEN, Dominic, 2950 Kapellen (BE); SCHWAB, Ekkehard, 67434 Neustadt (DE); MÜLLER, Jörn, 28832 Achim (DE); PENZEL, Ulrich, 01945 Tettau (DE); GEORGI, Gunter, Baton Rouge, LA 70817 (US); WEIDNER, Bernd, 01994 Wormlage (DE); TITTELBACH-HELMRICH, Dietrich, 01561 Tauscha (DE); DAHLHAUS, Jürgen, 33689 Dielefeld (DE)
(74) Vertreter: Isenbruck, Günter
(86) Internationale Anmeldenummer: PCT/EP2002/005423
(87) Internationale Veröffentlichungsnummer: WO 2002/094434

(56) Entgegenhaltungen:
- EP-A- 1 163 955
- DE-A- 19 909 177
- DE-A- 19 915 357
- DE-A- 19 922 038
- FR-A- 2 347 326
- US-A- 3 794 588
- US-A- 3 868 332

## Beschreibung

Die vorliegende Erfindung betrifft einen Hydrierkatalysator, der Nickel auf einem TiO₂₋Träger enthält. Der Katalysator wird insbesondere für die Hydrierung von nitroaromatischen Verbindungen zu entsprechenden aromatischen Aminoverbindungen eingesetzt.

Der Einsatz von nickelhaltigen Katalysatoren als Hydrierkatalysatoren ist seit langem bekannt. In Russian Journal of Applied Chemistry, Vol. 70, No. 8, 1997, Seiten 1236 bis 1253 ist die Herstellung derartiger nickelhaltiger Katalysatoren ausführlich beschrieben. Die beschriebenen Katalysatoren enthalten Nickel vornehmlich auf Siliciumdioxid oder Aluminiumoxid als Träger. Sie können dabei durch Fällung auf den Träger oder durch Copräzipitation hergestellt werden. Es werden auch SiO₂/TiO₂-Träger eingesetzt, wobei angegeben wird, daß TiO₂ als Modifizierungsmittel zu mechanisch stabilen Katalysatoren führen kann.

DD-A-152 065 betrifft ein Verfahren zur Hydrierung von Nitroaromaten mit rührstabilen Suspensionskatalysatoren. Der Katalysator wird durch Fällung einer Nickelsalzlösung in Gegenwart von Kieselgel mit einer bestimmten Komgrößenverteilung hergestellt.

DD-A-284 371 betrifft ein Verfahren zur Reduktion von Nitroaromaten in Gegenwart eines Ni/SiO₂-Katalysators. Der Katalysator wird dabei auf einem Träger mit einer Größe von mehr als 1 mm geträgert und nach der Aktivierung passiviert. Vor der Umsetzung wird der Katalysator im Reaktor vermahlen.

US 3,868,332 betrifft einen Hydrierkatalysator, der insbesondere zur Umwandlung von Benzol in Cyclohexan eingesetzt wird. Der Katalysator wird durch gemeinsame Fällung von Nickel- und Silikat-Ionen in Gegenwart eines porösen Siliciumdioxidträgers hergestellt.

WO 95/14647 betrifft ein Verfahren zur Oligomerisierung von Olefinen zu hochlinearen Oligomeren. Dabei wird ein oxidischer Katalysator eingesetzt, der Nickeloxid, Siliciumdioxid und Titandioxid sowie ein Alkalimetalloxid enthält. Der Katalysator wird durch gemeinsame Fällung einer Nickelsalzlösung mit einer Natronwasserglaslösung auf feinteiliges Titandioxidpulver hergestellt. Nickel liegt dabei in oxidischer Form vor, und der Katalysator wird in Form von gepreßten Tabletten als Festbett eingesetzt. Der Einsatz als Hydrierkatalysator wird nicht beschrieben.

DE-A-199 09 176 und DE-A-199 09 168 betreffen Hydrierkatalysatoren, die Nickel und häufig Zirkonium auf einem zirkoniumhaltigen Träger aufweisen.

Bei der Hydrierung von nitroaromatischen Verbindungen zu aromatischen Aminoverbindungen wird der Katalysator einem stark alkalischen Medium ausgesetzt. Ein im Reaktionsmedium dispergierter Katalysator wird bei einer Kreisführung des Reaktionsgemisches bzw. beim Rühren einer hohen mechanischen Beanspruchung ausgesetzt. Er sollte eine solche Partikelgrößenverteilung aufweisen, daß er unter Betriebsbedingungen sowohl gut dispergierbar als auch gut abtrennbar ist.

Aufgabe der vorliegenden Erfindung ist die Bereitstellung eines Katalysators für die Hydrierung von nitroaromatischen Verbindungen, der das vorstehende Eigenschaftsspektrum zeigt und die Nachteile der bekannten Katalysatoren vermeidet.

Die Aufgabe wird erfindungsgemäß gelöst durch einen Katalysator, enthaltend Nickel auf einem TiO₂-Träger, erhältlich durch gemeinsame Fällung von
- Nickel und
- mindestens einem weiteren Metall, ausgewählt aus Si, Zr, Hf, Erdalkalimetallen, Y, La, Ce und
- gegebenenfalls mindestens einem Dotiermetall, ausgewählt aus den Gruppen 5 bis 11 des Periodensystems der Elemente,
- aus einer Lösung, die entsprechende Metallsalze enthält, auf einen teilchenförmigen TiO₂-Träger, anschließendes Trocknen, Calcinieren und Reduzieren und gegebenenfalls Passivieren zum nickelhaltigen Katalysator.

Es wurde erfindungsgemäß gefunden, daß bei gemeinsamer Fällung von Nickel und einem anderen Trägerprekursormetall in Oxidform auf einen TiO₂-Träger teilchenförmige Katalysatoren erhalten werden, die sowohl in stark alkalischem Medium chemisch stabil sind als auch unter starker mechanischer Beanspruchung, beispielsweise durch eine Kreislaufpumpe physisch stabil sind. Ausgehend von üblichen teilchenförmigen TiO₂₋Trägem werden teilchenförmige Katalysatoren erhalten, die unter Betriebsbedingungen sowohl eine gute Dispergierbarkeit als auch eine gute Abtrennbarkeit aufweisen und einen guten Stofftransport erlauben.

Die gemeinsame Fällung von Nickel und einem weiteren Trägerprekursor erlaubt zudem eine vorteilhafte Teilchengrößenverteilung des Katalysators sowie die Ausbildung von kleinen Nickelkristalliten, die zu einer hohen Aktivität des Katalysators führen.

Die mittlere Teilchengröße (d₅₀) des Katalysators beträgt vorzugsweise mindestens 3 µm, besonders bevorzugt mindestens 4 µm, insbesondere mindestens 5 µm. Sie beträgt vorzugsweise maximal 80 µm, besonders bevorzugt maximal 40 µm.

Die Nickel-Kristallitgröße, bestimmt durch XRD, beträgt vorzugsweise mindestens 4 nm, besonders bevorzugt mindestens 7 nm. Sie beträgt vorzugsweise maximal 20 nm, besonders bevorzugt maximal 15 nm. Speziell bevorzugt sind Bereiche von 7 bis 15 nm und insbesondere 10 bis 12 nm. Diese Kristallitgrößen erlauben eine hohe Aktivität des Katalysators, wobei die Kristallite noch oberflächlich passiviert werden können, um den Katalysator an Luft handhabbar zu machen. Bei kleineren Teilchengrößen werden die Kristallite bei der Passivierung leichter durchoxidiert. Größere Kristallitgrößen vermindern die Aktivität des Katalysators bei einer gegebenen Nickelmenge.

Der Anteil an Nickel im erfindungsgemäßen Katalysator beträgt vorzugsweise 20 bis 80 Gew.-%, besonders bevorzugt 40 bis 70 Gew.-%, insbesondere 50 bis 65 Gew.-%, bezogen auf den gesamten Katalysator und bezogen auf Nickel als Metall.

Das Gewichtsverhältnis vom Oxid des mindestens einen weiteren Metalls zum TiO₂₋Träger beträgt vorzugsweise (0,2 bis 4):1, besonders bevorzugt (0,5 bis 2):1, insbesondere (0,8 bis 1,2):1. Speziell werden ähnliche Mengen an TiO₂-Träger und Oxid des mindestens einen weiteren Metalls (anderer Trägerprekursor) eingesetzt.

Sofern mindestens ein Dotiermetall eingesetzt wird, beträgt seine Menge, bezogen auf das Oxid und den gesamten Katalysator, 0,1 bis 10 Gew.-%, besonders bevorzugt 1 bis 7 Gew.-%.

Die erfindungsgemäßen Katalysatoren zeigen eine hohe mechanische Stabilität, so daß sie beim Betrieb als dispergierter Katalysator im Laufe des Einsatzes zwar eine Teilchengrößenverkleinerung erfahren, diese jedoch in einem Bereich bleibt, der die Abtrennung des Katalysators mit einem Settler erlaubt. Die Abtrennung über einen Settler vereinfacht die Trennung des Katalysators vom Reaktionsgemisch und die nachfolgende Rückführung des Katalysators.

Sofern geringere Mengen des Katalysators im Verlauf des Verfahrens Teilchengrößen kleiner der bevorzugten angegebenen Teilchengröße bilden, ist dieses nicht schädlich, da hierüber verbrauchter Katalysator aus dem Reaktionssystem ausgeschleust werden kann, da er nicht im Settler zurückgehalten wird.

Insbesondere die spezifische Trägerkombination gibt dem erfindungsgemäßen Katalysator das vorteilhafte Eigenschaftsspektrum.

Der erfindungsgemäße Katalysator kann nach dem Reduzieren auch passiviert werden, um ihn lagerfähig und an der Luft einfach handhabbar zu machen. Hierzu kann beispielsweise auf DE-A-199 09 175 verwiesen werden.

Dabei kann der Katalysator bei der Herstellung einem oder mehreren Formgebungsschritten unterworfen werden. Beispielsweise ist es möglich, den Katalysator nach dem Fällen und Trocknen, vor oder nach dem Calcinieren in eine gewünschte Form zu bringen, zu reduzieren, zu passivieren und sodann in Katalysatorteilchen der gewünschten Teilchengröße zu überführen. Beispielsweise kann der gefällte Katalysatorvorläufer aus der Fällösung abfiltriert und getrocknet werden, worauf Brocken aus dem Filterkuchen calciniert, reduziert, passiviert und wiederum vermahlen werden. Andere mögliche Formgebungsschritte sind die Tablettierung und das Strangpressen bzw. Extrusion.

Die Herstellung des erfindungsgemäßen Katalysators durch Fällung erfolgt allgemein vorzugsweise wie folgt:

TiO₂-Pulver (zum Beispiel S 150 von Kemira) wird auf einen Teilchendurchmesser < 100 µm gemahlen, in Wasser vorgelegt und 5 Minuten mit einem Ultrathurrax dispergiert. Gegebenenfalls wird jetzt Wasserglas als Si-Quelle zugegeben. Der pH-Wert wird eingestellt auf einen Wert zwischen 5 und 10, eine Lösung von Ni-Nitrat und gegebenenfalls zusätzlichen Salzen wie Zr-Acetat, Ce-Nitrat, Mg-Nitrat wird innerhalb von 30 min zugepumpt, und der pH wird durch gleichzeitiges Pumpen von einer Sodalösung und gegebenenfalls einer Wasserglaslösung bei einem Wert zwischen 5 und 10 konstant gehalten. Danach wird unter Einblasen von Luft 30 min nachgerührt. Der pH-Wert wird mit Sodalösung auf einen Wert von mindestens 7,5 gebracht. Das Fällprodukt wird abfiltriert und gewaschen, bis es nitratfrei ist. Der Filterkuchen wird getrocknet (12 h, 120°C) und anschließend calciniert (4 h, 400°C). Das so erhaltene Produkt wird auf einen mittleren Teilchendurchmesser von < 100 µm zerkleinert und 4 h bei 400 bis 550°C in einem Wasserstoffstrom reduziert.

Die Erfindung betrifft auch ein Verfahren zur Herstellung eines wie vorstehend definierten Katalysators, bei dem man die angegebenen Verfahrensschritte durchführt.

Zudem betrifft die Erfindung die Verwendung des Katalysators zur Hydrierung von nitroaromatischen Verbindungen.

Die Erfindung betrifft auch ein Verfahren zur Herstellung von aromatischen Aminoverbindungen durch Hydrierung entsprechender nitroaromatischer Verbindungen, wobei die Hydrierung in Gegenwart eines wie vorstehend definierten Katalysators durchgeführt wird.

Dabei können beliebige nitroaromatische Verbindungen eingesetzt werden. Vorzugsweise sind die nitroaromatischen Verbindungen ausgewählt aus Nitrobenzol, Nitrotoluol und Dinitrotoluol. Entsprechende herzustellende aromatische Aminoverbindungen sind Anilin, o-Toluidin, p-Toluidin und Toluylendiamin.

Die erfindungsgemäße Hydrierung kann in der Flüssig- und Gasphase kontinuierlich oder diskontinuierlich durchgeführt werden. Vorzugsweise wird sie in der Flüssigphase durchgeführt. Dabei kann die Umsetzung in Gegenwart eines geeigneten Lösungsmittels durchgeführt werden.

Besonders bevorzugt wird die Hydrierung in einer Dispersion des Katalysators durchgeführt.

Die Hydrierung wird vorzugsweise bei einer Temperatur im Bereich von 60 bis 200°C, besonders bevorzugt 100 bis 140°C und einem Wasserstoffdruck von 5 bis 100 bar, besonders bevorzugt 20 bis 30 bar durchgeführt.

Für eine Beschreibung der Verfahrensbedingungen kann auch auf DE-A-199 09 168 verwiesen werden.

Die Erfindung wird nachstehend anhand von Beispielen näher erläutert.

### Beispiele:

Die Herstellung der Katalysatoren erfolgte wie vorstehend im allgemeinen Herstellungsverfahren beschrieben. Zr wurde als Acetat, Si als Wasserglas, TiO₂ als Pulver eingesetzt, die anderen Metalle als Nitrate. Die gemeinsame Fällung erfolgt bei den in Tabelle 1 genannten pH-Werten, die gegebenenfalls durch Zusatz von Sodalösung angepaßt wurden. Es wurde dann unter Einblasen von Luft für 1 h bei Raumtemperatur nachgerührt, worauf der pH-Wert, falls erforderlich durch Zugabe von Sodalösung, auf pH = 7,5 eingestellt wurde. Nach Abnutschen der Fällung und Waschen mit Wasser zur Entfernung von Nitrat wurde für 12 h bei 120°C getrocknet, gegebenenfalls durch ein 1 mm Sieb gedrückt, und für 4 h bei Temperaturen im Bereich von 350 bis 550°C reduziert und passiviert.

Die nach dem vorstehenden Verfahren hergestellten Katalysatoren wurden mittels XRD zur Bestimmung der Nickel-Kristallitgröße untersucht. Zudem wurde der d₅₀-Wert aus der Partikelgrößenverteilung bestimmt. Dies kann beispielsweise in einer Sympatec-Helos Suspensionszelle erfolgen, wobei als Dispersionsmittel Wasser mit 1 g/l Natriumpyrophosphat eingesetzt wird. Zur Bestimmung der mechanischen Stabilität der Katalysatoren wurden diese für 1, 3 oder 10 min im Ultraturrax gemahlen. Sofern bei unterschiedlichen Temperaturen reduziert wurde, sind die Temperaturen angegeben. Die Ergebnisse sind in der nachstehenden Tabelle 1 zusammengefaßt.

**Tabelle 1 Katalysatoren, hergestellt durch gemeinsames Auffällen, mit pH-Wert der Fällung, Ni-Kristallitgröße (XRD) und d₅₀-Wert aus der Partikelgrößenverteilung nach 1/3/10 min Mahlen im Ultraturrax**

| **Kat.** | **Herstellung** | **Ni (nm)** | **d**_{**50**} **(µm)** |
|---|---|---|---|
| 1 | Fällung 50% Ni auf 25% ZrO₂/25% TiO₂ RT, pH 5,7 | 14 | (350°C) 8/6,4 / 4,6,(450°C) 8,1/6,4/5,7 |
| 2 | Fällung 50% Ni auf 15% ZrO₂/35% TiO₂ RT, pH 5,7 | 21 | 7,5/4,5/3 |
| 3 | Fällung 50% Ni auf 25% TiO₂/25% ZrO₂, pH 5 | | 8/6/3,5 |
| 4 | Fällung 50% Ni auf 45% TiO₂+5% Ce₂O₃ pH 6 | 12,5 | 7,3/4/3,7 |
| 5 | Fällung 50% Ni auf 25% TiO₂+25% MgO pH 9 | 18,5 | 18/7,5/3,7 |
| 6 | Fällung 50% Ni auf 25% TiO₂+25% MgO pH 10 | 6,0 | 18/12,5/3,4 |
| 7 | Fällung 50% Ni auf 25% TiO₂+25% SiO₂ pH 6 | 4,5 | |
| 8 | Fällung 50% Ni auf 25% TiO₂+25% SiO₂ pH 9 | 5,0 | 43/40/13 |
| 9 | Fällung 50% Ni auf 25% TiO₂+25% SiO₂ pH 6 | 5,5 | 55/40/17 |
| 10 | Fällung 50% Ni auf 25% TiO₂+25% MgO:SiO₂ pH 9 | 6,5 | 30/25/15 |
| 11 | Fällung 50% Ni auf 25% TiO₂+25% MgO:SiO₂ pH 10 | 6,0 | 42/33/20 |
| 12 | Fällung 50% Ni auf 25% TiO₂+25% MgO:ZrO₂ pH 9 | 9,5 | 18/14/3,4 |
| 13 | Fällung 50% Ni auf 25% TiO₂+25% MgO:ZrO₂ pH 10 | 6,0 | 36/31/8 |
| 14 | Fällung 50% Ni auf +15% ZrO₂+5% Ce₂O₃ auf 30% TiO₂ pH 5,7 | 10 | 4,4/4,1/3,8 |
| 15 | Fällung 50% Ni+25% SiO₂ auf 25% TiO₂ pH 5,7 | 5,5 | 10,6/8,8/3,0 |
| 16 | Fällung 50% Ni+5% Fe+22,5% MgO:SiO₂ auf 22,5% TiO₂ pH 9 | 6 | 29/18/10 |
| 17 | Fällung 50% Ni+5% Fe+22,5%ZrO₂ auf 22,5% TiO₂ pH 5,6 | 15 | 7,6/6,6/ 5,6 |
| 18 | Fällung 70% Ni+15% MgO:SiO₂ auf 15% TiO₂ pH 9 | 6,5 | 7,4/4,4/3 |
| V | 1 Fällung 50% Ni auf 50% TiO₂ pH 5,6 | 54 | 5,6/4,8/4,3 |

| | | | |
|---|---|---|---|
| RT: Raumtemperatur | | | |

Es wurden jeweils die von TiO₂ verschiedenen Komponenten auf TiO₂ gefällt. Die Prozentangaben beziehen sich auf das Oxidgewicht, für Ni auf das Metallgewicht

Die Katalysatoren wurden zur Hydrierung von Dinitrotoluol (DNT) zu Toluylendiamin (TDA) eingesetzt. Dabei wurde wie folgt vorgegangen:

Ein 300 ml Autoklav wurde mit 0,8 g eines frisch reduzierten Katalysators, 100 ml n-Butanol, 20 g DNT befüllt, mit Wasserstoff auf 25 bar hochgepresst und auf 80°C erhitzt. Als Maß für die Aktivität wurde die Wasserstoffaufnahme in 1/min genommen.

Die Ergebnisse der Abreicherungsversuche sind in Tabelle 2 zusammengefaßt.

**Tabelle 2 Abreicherungsversuche (80°C, 25 bar, 100 ml n-Butanol, 20 g DNT, 0,8 g Katalysator, 3h)**

| **Kat.** | **Umsatz (5)** | **TDA-Ausb. (%)** | **H**_{**2**}**-Aufn. (l/min)** |
|---|---|---|---|
| 1 | 100 | 96,76 | 0,10 |
| 2 | 100 | 97,64 | 0,40 |
| 7 | 100 | 98,78 | 0,70 |
| 8 | 100 | 99,03 | 0,48 |
| 9 | 100 | 99,11 | 0,37 |
| 10 | 100 | 97,35 | 0,40 |
| 11 | 100 | 98,09 | 0,67 |
| 12 | 100 | 97,77 | 0,12 |
| 13 | 100 | 97,79 | 0,17 |
| 14 | 100 | 99,21 | 0,23 |
| 15 | 100 | 97,58 | 0,75 |
| 16 | 100 | 98,6 | 2,00 |
| 17 | 100 | 96,82 | 0,28 |
| 18 | 100 | 99 | 0,53 |
| V1 | 100 | 99,48 | 0,13 |

Aus den Ergebnissen geht hervor, daß die erfindungsgemäßen Katalysatoren eine vorteilhafte Kombination von Aktivität und mechanischer Stabilität aufweisen.

## Patentansprüche

1. Katalysator, enthaltend Nickel auf einem TiO₂-Träger, erhältlich durch gemeinsame Fällung von
- Nickel und
- mindestens einem weiteren Metall, ausgewählt aus Erdalkalimetallen, Y, La, Ce und
- gegebenenfalls mindestens einem Dotiermetall, ausgewählt aus den Gruppen 5 bis 11 des Periodensystems der Elemente,
- aus einer Lösung, die entsprechende Metallsalze enthält auf einen teilchenformigen TiO₂-Träger, anschließendes Trocknen, Calcinieren und Reduzieren und gegebenenfalls Passivieren zum nickelhaltigen Katalysator.

2. Katalysator nach Anspruch 1, **dadurch gekennzeichnet, dass** der Anteil an Nickel im Katalysator 20 bis 80. Gew.-%, bezogen auf den gesamten Katalysator, beträgt.

3. Katalysator nach Anspruch 1 oder 2, **dadurch gekennzeichnet, dass** das Gewichtsverhältnis vom Oxid des mindestens einen weiteren Metalls zum TiO₂₋Träger (0,2 bis 4): 1 beträgt.

4. Katalysator nach einem der Ansprüche 1 bis 3, **dadurch gekennzeichnet, daß** die Nickel-Kristallitgröße 4 bis 20 nm beträgt.

5. Katalysator nach einem der Ansprüche 1 bis 4, **dadurch gekennzeichnet, dass** die mittlere Teilchengröße (d₅₀) des Katalysators 3 bis 80 µm beträgt.

6. Katalysator nach einem der Ansprüche 1 bis 5, **dadurch gekennzeichnet, dass** der Katalysator nach dem Reduzieren passiviert wird.

7. Verfahren zur Herstellung eines Katalysators gemäß einem der Ansprüche 1 bis 6, **dadurch gekennzeichnet, dass** man die angegebenen Verfahrensschritte durchführt.

8. Verwendung eines Katalysators gemäß einem der Ansprüche 1 bis 6 zur Hydrierung von nitroaromatischen Verbindungen.

9. Verfahren zur Herstellung von aromatischen Ammoverbindungen durch Hydrierung entsprechender nitroaromatischer Verbindungen, **dadurch gekennzeichnet, dass** die Hydrierung in Gegenwart eines Katalysators durchgeführt wird, wie er in einem der Ansprüche 1 bis 6 definiert ist.

10. Verfahren nach Anspruch 9, **dadurch gekennzeichnet, dass** die aromatischen Aminoverbindungen ausgewählt sind aus Anilin, o-Toluidin, p-Toluidin und Toluylendiamin.

## Claims

1. A catalyst comprising nickel on a TiO₂ support and obtainable by coprecipitation of
- nickel and
- at least one further metal selected from among alkaline earth metals, Y, La and Ce, and
- optionally at least one dopant metal selected from groups 5 to 11 of the Periodic Table of the Elements,
- from a solution which comprises the corresponding metal salts onto a particulate TiO₂ support, subsequent drying, calcination and reduction and optionally passivation to give the nickel-containing catalyst.

2. A catalyst as claimed in claim 1, wherein the proportion of nickel in the catalyst is from 20 to 80% by weight, based on the total catalyst.

3. A catalyst as claimed in claim 1 or 2, wherein the weight ratio of the oxide of the further metal or metals to the TiO₂ support is (0.2 - 4):1.

4. A catalyst as claimed in any of claims 1 to 3, wherein the nickel crystallite size is from 4 to 20 nm.

5. A catalyst as claimed in any of claims 1 to 4, wherein the mean particle size (d₅₀) of the catalyst is from 3 to 80 µm.

6. A catalyst as claimed in any of claims 1 to 5, which is passivated after reduction.

7. A process for preparing a catalyst as claimed in any of claims 1 to 6, which comprises carrying out the process steps indicated.

8. The use of a catalyst as claimed in any of claims 1 to 6 for the hydrogenation of nitroaromatic compounds.

9. A process for preparing aromatic amino compounds by hydrogenation of corresponding nitroaromatic compounds, wherein the hydrogenation is carried out in the presence of a catalyst as defined in any of claims 1 to 6.

10. A process as claimed in claim 9, wherein the aromatic amino compounds are selected from among aniline, o-toluidine, p-toluidine and toluenediamine.

## Revendications

1. Catalyseur contenant du nickel sur un support de TiO₂, qu'on obtient par précipitation commune :
- de nickel et
- d'au moins un autre métal choisi parmi les métaux alcalino-terreux, le Y, le La, le Ce et
- le cas échéant d'au moins un métal de dopage sélectionné parmi les groupes 5 à 11 du système périodique des éléments,
- d'une solution qui contient les sels de métal correspondants, sur un support de TiO₂ en forme de particule, séchage subséquent, calcination et réduction et le cas échéant passivation en catalyseur contenant du nickel.

2. Catalyseur selon la revendication 1, **caractérisé en ce que** la proportion de nickel dans le catalyseur est de 20 à 80% en poids, sur base du catalyseur total.

3. Catalyseur selon la revendication 1 ou 2, **caractérisé en ce que** le rapport pondéral d'oxyde du au moins un autre métal, à support de TiO₂ est de (0,2 à 4):1.

4. Catalyseur selon l'une quelconque des revendications 1 à 3, **caractérisé en ce que** la grosseur des cristallites de nickel est de 4 à 20 nm.

5. Catalyseur selon l'une quelconque des revendications 1 à 4, **caractérisé en ce que** la grosseur moyenne de particule (d₅₀) du catalyseur est de 3 à 80 *µ*m.

6. Catalyseur selon l'une quelconque des revendications 1 à 5, **caractérisé en ce que** le catalyseur est passivé après la réduction.

7. Procédé de fabrication d'un catalyseur selon l'une quelconque des revendications 1 à 6, **caractérisé en ce qu'**on réalise les étapes de procédé données.

8. Utilisation d'un catalyseur selon l'une quelconque des revendications 1 à 6 pour l'hydrogénation de composés nitroaromatiques.

9. Procédé de préparation de composés amino aromatiques par hydrogénation des composés nitroaromatiques correspondants, **caractérisé en ce que** l'hydrogénation s'effectue en présence d'un catalyseur tel que défini dans l'une quelconque des revendications 1 à 6.

10. Procédé selon la revendication 9, **caractérisé en ce que** les composés amino aromatiques sont sélectionnés parmi l'aniline, la o-toluidine, la p-toluidine et la toluylènediamine.
